# DEMANDE DE BREVET EUROPEEN

(11) **EP 2 277 857 A2**
(43) Date de publication de la demande: **26.01.2011**
(21) Numéro de dépôt: 10011469.3
(22) Date de dépôt: 02.08.2006
(51) Int. Cl.: C07C 233/18, C07C 231/24, A61K 31/165, A61P 25/00, A61P 37/00, A61P 15/08, A61P 9/00, A61P 3/00

(54) **Nouvelle forme cristalline V de l'agomélatine, son procédé de préparation et les compositions pharmaceutiques qui la contiennent**

(30) Priorité: 03.08.2005 FR 0508278
(62) Demande divisionnaire de: 06291251.4
(71) Demandeur: Les Laboratoires Servier, 92284 Suresnes Cedex (FR)
(72) Inventeur: Coquerel, Gérard, 76520 Boos (FR); Linol, Julie, 76100 Rouen (FR); Souvie, Jean-Claude, 64000 Pau (FR)

(57) **Abrégé**

Forme cristalline V du composé de formule (I) : 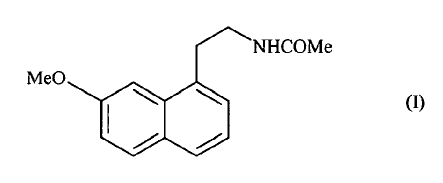 caractérisée par son diagramme de diffraction X sur poudre.

## Description

La présente invention concerne une nouvelle forme cristalline V de l'agomélatine ou *N*-[2-(7-méthoxy-1-naphtyl)éthyl]acétamide de formule (I) : 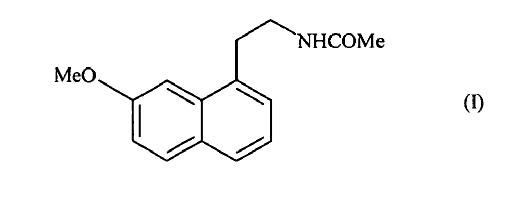 son procédé de préparation ainsi que les compositions pharmaceutiques qui la contiennent.

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confère une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte tenu de l'intérêt pharmaceutique de ce composé, il était primordial de l'obtenir avec une excellente pureté, et notamment sous une forme parfaitement reproductible, présentant des caractéristiques intéressantes de dissolution et de facilité de formulation permettant son stockage prolongé sans conditions particulières de température, de lumière, d'humidité ou de taux d'oxygène.

Le brevet EP 0 447 285 décrit l'accès en huit étapes à l'agomélatine à partir de la 7-méthoxy-1-tétralone. Cependant, ce document ne précise pas les conditions d'obtention de l'agomélatine sous une forme présentant ces caractéristiques de manière reproductible.

La demanderesse a présentement mis au point un procédé d'obtention de l'agomélatine sous une forme cristalline bien définie, parfaitement reproductible et présentant de ce fait des caractéristiques intéressantes de dissolution et de facilité de formulation.

Plus spécifiquement, la présente invention concerne la forme cristalline V du composé de formule (I), caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°) exp.** | **d (Å) exp.** | **Intensité (%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

L'invention s'étend également au procédé de préparation de la forme cristalline V du composé de formule (I), caractérisé en ce que l'on soumet l'agomélatine à un broyage mécanique dit « de haute énergie ».
Dans le procédé de cristallisation selon l'invention, on peut utiliser le composé de formule (I) obtenu par n'importe quel procédé.

L'invention s'étend également à un autre procédé de préparation de la forme cristalline V du composé de formule (I), caractérisé en ce que l'on chauffe l'agomélatine jusqu'à fusion complète puis on le place à température ambiante et simultanément on ajoute une très petite quantité de la forme cristalline V du composé de formule (I) fraîchement préparée, puis on laisse ensuite refroidir jusqu'à cristallisation complète.
De préférence, dans ce second procédé de cristallisation selon l'invention, l'agomélatine sera fondu à 110°C.
La quantité de forme cristalline V ajoutée dans ce second procédé de cristallisation selon l'invention sera comprise de préférence entre 1/100 et 1/50 du poids d'agomélatine.
Dans ce second procédé de cristallisation selon l'invention, on peut utiliser le composé de formule (I) obtenu par n'importe quel procédé.

L'obtention de cette forme cristalline a pour avantage de permettre la préparation de formulations pharmaceutiques ayant une composition constante et reproductible, présentant des caractéristiques de dissolution particulièrement intéressantes, ce qui est particulièrement avantageux lorsque ces formulations sont destinées à l'administration orale.

L'étude pharmacologique de la forme V ainsi obtenue a montré une importante activité sur le système nerveux central ainsi que sur la microcirculation qui permet d'établir son utilité dans le traitement du stress, des troubles du sommeil, de l'anxiété, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, la forme V de l'agomélatine peut être utilisée dans les dysfonctionnements sexuels, qu'elle possède des propriétés d'inhibiteurs de l'ovulation, d'immunomodulateurs et qu'elle est susceptible d'être utilisée dans le traitement des cancers.

La forme cristalline V de l'agomélatine sera utilisée de préférence dans les traitements de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'invention s'étend aussi aux compositions pharmaceutiques renfermant comme principe actif la forme cristalline V du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,1 mg à 1 g par jour en une ou plusieurs prises.

Les exemples ci-dessous illustrent l'invention, mais ne la limitent en aucune façon.

### Exemple 1 : Forme cristalline V du N-[2-(7-Méthoxy-1-naphtyl)éthyl]acétamide

100 g de *N*-[2-(7-Méthoxy-1-naphtyl)éthyl]acétamide sont placés dans le broyeur mécanique de type vario-planetary mill pendant environ 6 heures et le solide obtenu est caractérisé par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°) exp.** | **d (Å) exp.** | **Intensité (%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

### Exemple 2 : Forme cristalline V du N-[2-(7-Méthoxy-1-naphtyl)éthyl]acétamide

4 g de *N*-[2-(7-Méthoxy-1-naphtyl)éthyl]acétamide sont placés dans une étuve ventilée à 110°C. Après une heure à 110°C, le produit est mis à température ambiante et ensemencé avec 0,05 g de forme cristalline V du *N*-[2-(7-Méthoxy-1-naphtyl)éthyl]acétamide structuralement pure obtenue par broyage haute énergie. Au bout de 5 minutes, la cristallisation est totale et le solide obtenu est caractérisé par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°) exp.** | **d (Å) exp.** | **Intensité (%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

### Exemple 3 : Composition pharmaceutique

Formule de préparation pour 1000 comprimés doses à 25 mg :

| | |
|---|---|
| Composé de l'Exemple 1 ou 2 | 25 g |
| Lactose monohydrate | 62 g |
| Stéarate de Magnésium | 1,3 g |
| Amidon de maïs | 26 g |
| Maltodextrines | 9 g |
| Silice colloïdale anhydre | 0,3 g |
| Amidon de maïs prégélatinisé type A | 4 g |
| Acide stéarique | 2,6 g |

### Exemple 4 : Composition pharmaceutique

Formule de préparation pour 1000 comprimés doses à 25 mg :

| | |
|---|---|
| Composé de l'Exemple 1 ou 2 | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

## Revendications

1. Forme cristalline V de l'agomélatine de formule (I) : 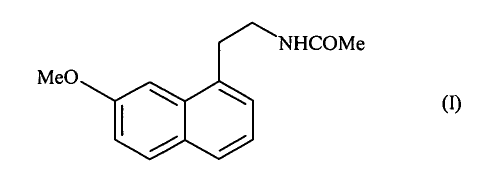 **caractérisée par** le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :
| **2-Theta (°) exp.** | **d (À) exp.** | **Intensité (%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

2. Procédé de préparation de la forme cristalline V du composé de formule (I) selon la revendication 1, **caractérisé en ce que** l'on l'on soumet l'agomélatine à un broyage mécanique dit « de haute énergie ».

3. Procédé de préparation de la forme cristalline V du composé de formule (I) selon la revendication 1, **caractérisé en ce que** l'on l'on chauffe l'agomélatine jusqu'à fusion complète puis on le place à température ambiante et simultanément on ajoute une très petite quantité de la forme cristalline V du composé de formule (I) fraîchement préparée, puis on laisse ensuite refroidir jusqu'à cristallisation complète.

4. Compositions pharmaceutiques contenant comme principe actif la forme cristalline V de l'agomélatine selon la revendication 1, en combinaison avec un ou plusieurs véhicules inertes, non toxiques et pharmaceutiquement acceptables.

5. Compositions pharmaceutiques selon la revendication 4 utiles pour la fabrication de médicaments pour traiter les troubles du système mélatoninergique.

6. Compositions pharmaceutiques selon la revendication 4 utiles pour la fabrication de médicaments pour le traitement des troubles du sommeil, du stress, de l'anxiété, des dépressions saisonnières ou de la dépression majeure, des pathologies cardiovasculaires, des pathologies du système digestif, des insomnies et fatigues dues aux décalages horaires, de la schizophrénie, des attaques de paniques, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, des troubles de la circulation cérébrale, ainsi que dans les dysfonctionnements sexuels, en tant qu'inhibiteurs de l'ovulation, d'immunomodulateurs et dans le traitement des cancers.
